Europäisches Patentamt

European Patent Office

Office européen des brevets

(11) Numéro de publication: **0 224 463**
**A1**

(12) # DEMANDE DE BREVET EUROPEEN

(21) Numéro de dépôt: 86870155.8

(22) Date de dépôt: **24.10.86**

(51) Int. Cl.⁴: **C 12 Q 1/34**
**C 12 Q 1/68**

(30) Priorité: 25.10.85 FR 8515882
18.04.86 FR 8605635

(43) Date de publication de la demande:
**03.06.87 Bulletin 87/23**

(84) Etats contractants désignés:
**AT BE CH DE ES FR GB GR IT LI LU NL SE**

(71) Demandeur: **N.V. SOPAR S.A.**
**Rue Ducale, 29**
**B-1000 Bruxelles (BE)**

(72) Inventeur: **Taper, Henryck**
**Rue Dray, 12**
**B-5992 Nodebais (BE)**

**Lans, Marc**
**Chaussée de Groenendael, 127**
**B-1990 Hoeilaart (BE)**

**Roberfold, Marcel**
**Avenue Marie-José, 169**
**B-1200 Bruxelles (BE)**

(74) Mandataire: **Duyck, Frans N.E. et al**
**OFFICE KIRKPATRICK Square de Meeûs, 4**
**B-1040 Bruxelles (BE)**

(54) **Procédé de détermination de l'activité des DNAases.**

(57) Procédé détermination de l'activité des DNAases dans les liquides biologiques.

On ajoute le liquide biologique à examiner à un milieu de réaction composé d'une solution tampon, d'un sel soluble de magnésium, d'un sel soluble de calcium et d'acide désoxyribo-nucléique; on maintient le mélange ainsi obtenu pendant 45 à 90 minutes à une température de 40 à 63°C, puis on le dénature, on sépare les substances insolubles et on soumet le liquide restant à une mesure spectrophotométrique à 260 nm par rapport à une solution de référence.

Le procédé est applicable notamment au diagnostic du cancer et à l'évaluation de l'efficacité des thérapeutiques appliquées à son traitement.

EP 0 224 463 A1

**Description**

Procédé de détermination de l'activité des DNAases.

La présente invention concerne un procédé de détermination de l'activité du complexe d'enzymes désoxyribonucléases (DNAases) dans les liquides biologiques, cette détermination trouvant son application notamment au diagnostic du cancer, à l'évaluation de l'efficacité des thérapies appliquées à son traitement ainsi qu'à la détection d'une reprise tumorale (métastases).

L'invention sera décrite ci-après en se référant aux figures annexées, dans lesquelles :

- Fig. 1 est la distribution des DNAases alcalines dans le sérum des sujets sains et des patients cancéreux non traités;

- Fig. 2 est la représentation synthétique de l'évolution de l'activité des désoxyribonucléases par rapport à la réponse clinique;

- Fig. 3 est le profil d'évolution de l'activité des DNAases alcalines telle que déterminée par le procédé suivant la présente invention chez un patient cancéreux traité par chimiothérapie.

Il a été constaté que l'activité des DNAases dans les liquides biologiques est modifiée chez des sujets porteurs de tumeurs malignes (par rapport à cette activité des DNAases chez des sujets sains).

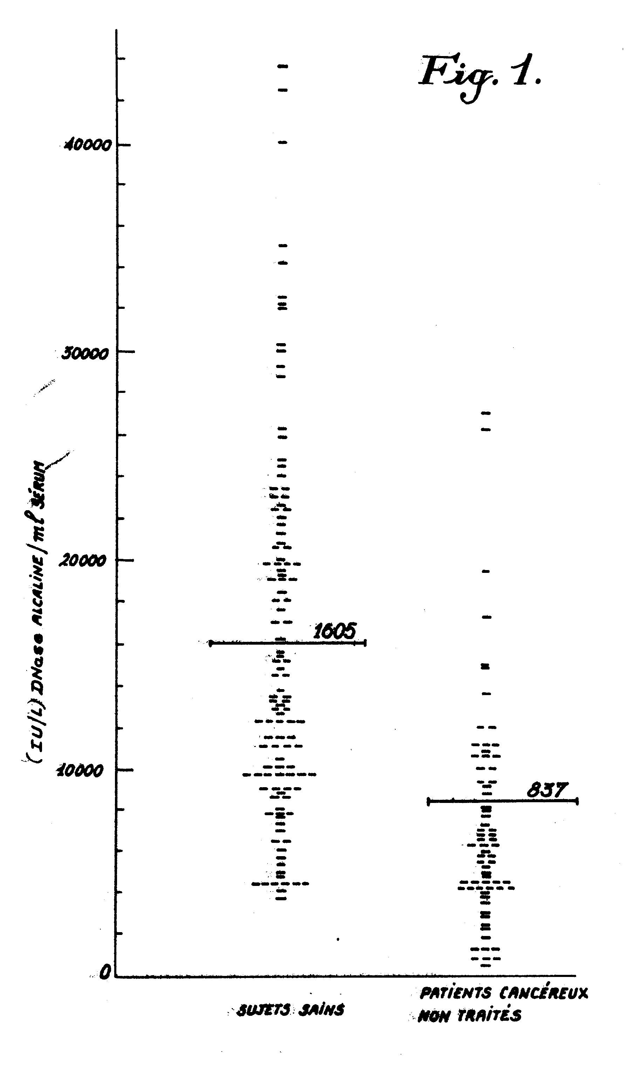

C'est ainsi qu'à la Fig. 1, on observe que la moyenne d'activité sérique des DNAases chez les patients porteurs de tumeurs malignes est significativement ($p < 0,01$) plus basse que la moyenne observée chez les personnes saines.

Il a également été constaté que le traitement des patients cancéreux peut provoquer un changement de l'activité des DNAases dans leurs liquides biologiques.

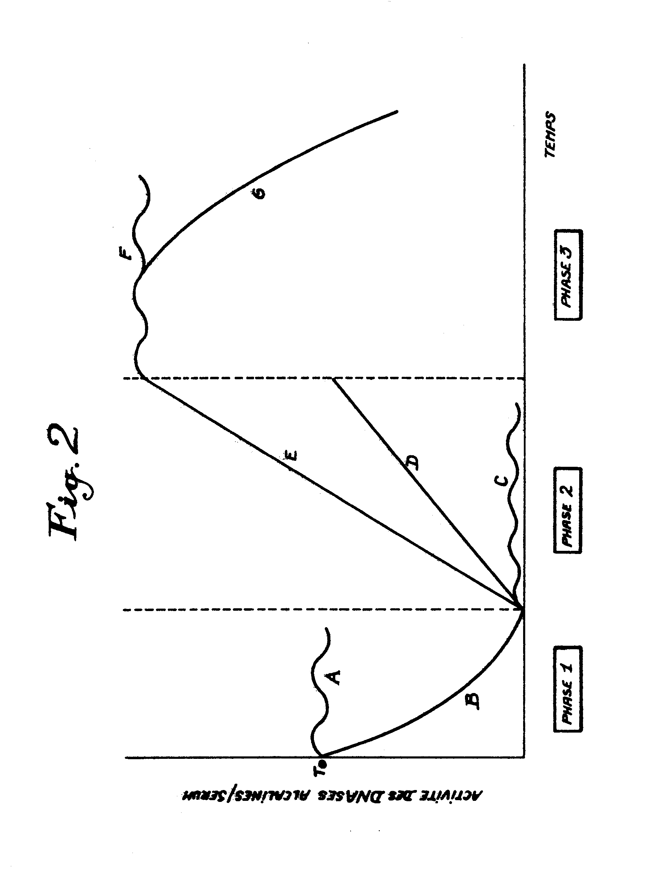

Le profil d'évolution de l'activité des DNAases selon l'efficacité du traitement et la réponse de la tumeur est donné dans la Fig. 2 en annexe.

Ainsi que ce schéma le montre, on y distingue plusieurs phases. Dans la phase I couvrant les jours qui suivent le début d'un traitement thérapeutique de patients cancéreux, on n'observe pas de changement de l'activité sérique des DNAases (courbe A) dans le cas de tumeurs résistant au traitement, tandis que lorsque la réaction au traitement est positive, on constate une nette diminution de l'acitivité sérique des DNAases (courbe B).

La phase II s'étale sur les semaines qui suivent le traitement. Si, après une réponse initiale au traitement, l'activité n'augmente pas à nouveau, l'évolution est très défavorable et se termine très souvent par une fatale progression de la tumeur (courbe C).

Si, après une réponse initiale au traitement, l'activité augmente, mais ne dépasse pas la valeur avant traitement, on observe une régression partielle de la tumeur (courbe D).

Enfin, si après une réponse initiale au traitement, l'activité augment à nouveau au point de dépasser la valeur d'origine, on observe une régression complète de la tumeur (courbe E).

Il a également été observé que l'activité des DNAases pouvait décroître en absence de traitement chez des patients qui avait été soignés et chez lesquels on avait diagnostiqué une régression complète. Ce phénomène est montré dans la phase III de la Fig. 2 qui s'étale sur les mois qui suivent le traitement. Tant que la régression demeure complète (courbe F), l'activité est constante. Cependant, lors d'une reprise de la tumeur, l'activité chute (courbe G).

Toutes ces variations d'activité ne peuvent cependant être décelées qu'en utilisant le procédé suivant l'invention.

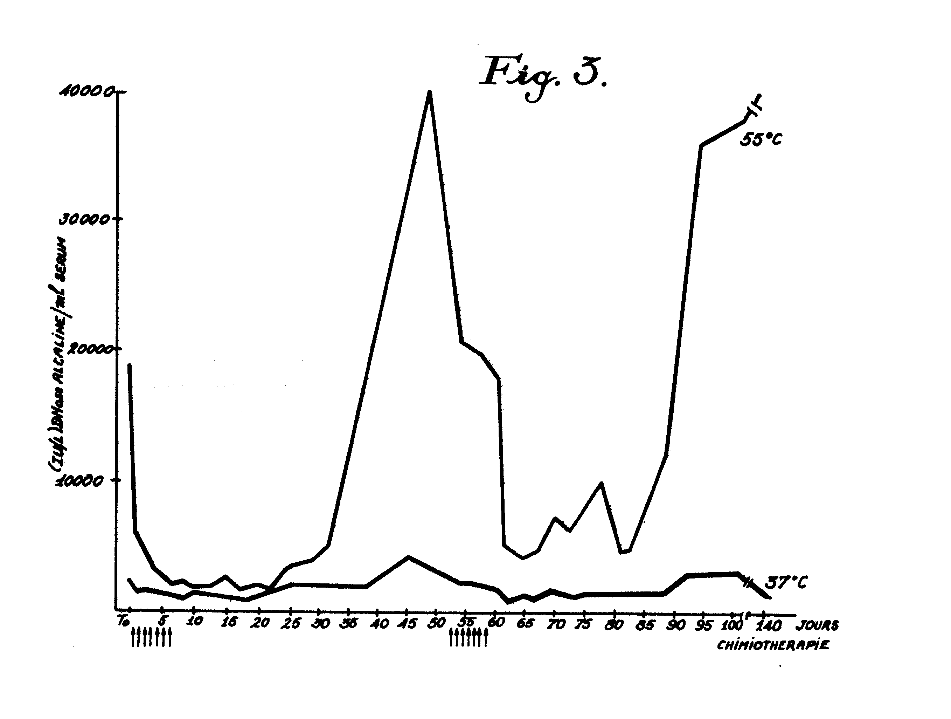

La Fig. 3 montre deux courbes analogues pour un patient cancéreux traité par deux traitements successifs de chimiothérapie. Une de ces deux courbes indiquée "55° C" montre l'évolution de l'activité des DNAases alcalines telle que déterminée par le procédé suivant l'invention. L'autre courbe indiquée "37° C", qui est donnée à titre de comparaison, utilise une procédure classique et sera commentée plus loin dans le présent mémoire descriptif.

Ainsi que le montre la Fig. 3, seul le procédé suivant l'invention permet d'obtenir les informations biologiques en corrélation avec l'évolution de la tumeur suite au traitement.

En disposant donc d'une méthode fiable et sensible permettant de mesurer l'activité des DNAases, on peut dépister l'existence d'une tumeur et vérifier si tel traitement est efficace ou non et, dans l'affirmative, suivre son déroulement et son degré de succès. Les variations d'activité enzymatique qui suivent directement le traitement peuvent donc donner des indications précieuses pour établir ou corriger la thérapie pour chaque patient individuellement.

Pour qu'une telle méthode de mesure de l'activité des DNAases puisse être efficace, il faudrait qu'elle soit 1) suffisamment sensible pour déterminer de manière probante le niveau d'activité des DNAases chez l'homme, 2) qu'elle soit parfaitement reproductible et 3) qu'elle soit simple au point de pouvoir être utilisée par le personnel habituel d'un laboratoire d'analyse médicale, 4) qu'elle mette en évidence une activité DNAases qui puisse être corrélée avec l'évolution de la tumeur suite au traitement.

Les procédés connus pour la détermination de l'activité des DNAases ne remplissent pas l'ensemble de ces conditions. Deux procédés se distinguent par leur relative simplicité et sont basés sur la mesure de l'extinction spectrophotométrique à 260 nm des polynucléotides solubles en milieu acide libérés par l'hydrolyse du DNA, à savoir:

a) la méthode de Loiselle et Carrier (Rev. Can. Biol., 22, 341-346 (1963)).

On ajoute 0,5 ml d'un sérum à examiner à 0,3 ml d'une solution à 0,4% de DNA (de sperme de saumon) dans 2 ml d'une solution tampon de pH 7,6 (Tris-HCl, 0,05 M) et de 0,1 ml d'une solution de chlorure de magnésium (0,342 M) et de chlorure de calcium (0,0162 M), ensuite on dilue avec une solution de sucrose à 0,35 M pour obtenir 3,6 ml. Après une incubation de 3 heures, à 37°C, on y ajoute 1,2 ml d'acide perchlorique à 25% (ce qui correspond à 4 M), et après 10 minutes, on centrifuge la solution à 2800 g. On mesure l'extinction de la solution à 260 nm.

b) la méthode de Spandidos et al. (Europ. J. Cancer, 16, 1615-1616 (1980)).

On dilue 100 µg de DNA (de thymus de veau) dans une solution tampon à pH 8 (Tris-HCl). On y ajoute 25 µl du sérum à examiner, et on maintient la solution pendant 15 minutes à 25°C. Ensuite, on y ajoute 2 ml d'acide perchlorique (1,5 M) à 4°C et on centrifuge la solution à 3000 g. L'extinction de la solution est mesurée à 260 nm.

Ces deux procédés ne sont malheureusement pas suffisamment sensibles.

Ainsi, pour la méthode de Loiselle et Carrier, malgré un temps d'incubation de 3 heures, un volume important de sérum, une quantité important de DNA il arrive souvent qu'on ne parvient pas à mettre en évidence l'activité enzymatique.

D'autre part, la méthode de Spandidos utilise une quantité faible de DNA, un volume faible de sérum, pas d'ions ajoutés et l'incubation dure 15 minutes seulement à 25°C. Mais, cette méthode donne souvent des valeurs négatives (par rapport au test blanc) et non reproductibles, le même échantillon donnant des très grandes variations de densité optique d'une mesure à l'autre.

Le fait que l'activité n'est pas décelée par la méthode Spandidos est probablement dû à l'absence d'ions dans le milieu d'incubation, ce qui diminue de manière substantielle l'activité enzymatique. En effet, il est connu que l'ion Mg++ active l'enzyme et que l'ion Ca++ potentialise cette action.

Le but de l'invention est d'offrir un procédé simple, reproductible, à sensibilité élevée et universellement applicable, pour déterminer l'activité des DNAases dans des liquides biologiques.

La présente invention a pour objet un procédé de détermination de l'activité des désoxyribonucléases dans des liquides biologiques, procédé suivant lequel on ajoute le liquide biologique à examiner à un milieu de réaction composé d'une solution tampon, d'acide désoxyribonucléique d'un sel soluble de magnésium et d'un sel soluble de calcium. On maintient le mélange de réaction ainsi obtenu pendant un temps fixe prédéterminé, de préférence pendant 45 à 90 minutes, avantageusement pendant 50 à 70 minutes, à une température de 40 à 63 °C, de préférence à une température de 55 à 60°C. Ensuite, on dénature le mélange, on sépare les substances insolubles du mélange de réaction et on soumet le liquide restant à une mesure spectrophotométrique à 260 nm par rapport à une solution de référence.

Après avoir dénaturé le mélange, il est avantageux de le refroidir par exemple à une température proche de 0°C, et de le maintenir à cette température pendant au moins 30 minutes avant de séparer les substances insolubles.

On soumet le liquide restant à une mesure spectrophotométrique à 260 nm par rapport à une solution de référence (blanc) préparée dans les mêmes conditions opératoires, mais non incubée ou à laquelle on ajoute un inhibiteur des DNAases tel que l'EDTA (acide édétique) qui bloque l'activité enzymatique.

Une différence technique essentielle par rapport aux procédés connus est l'incubation à une température de 40 à 63°C, idéalement de 55 à 60°C, pendant un temps de réaction fixe prédéterminé de préférence pendant 45 à 90 minutes. L'effet favorable de cette température relativement élevée paraît à première vue facile à concevoir, car une augmentation de la température donne une augmentation de la vitesse de réaction et une meilleure utilisation de la réactivité des ingrédients.

En fait, ceci est exact en ce qui concerne les réactions chimiques normales, mais ne peut pas être appliqué tel quel aux réactions biochimiques faisant intervenir les protéines telles que les enzymes. Les réactions impliquant des enzymes se déroulent d'habitude à la température ambiante ou physiologique, en quelques minutes et, en général, ne peuvent supporter les températures au-dessus de 40°C. Il en ressort que la température utilisée pour l'invention est supérieure à la température habituelle. En effet, les températures de réaction du procédé de l'invention se trouvent dans une zone où, normalement, les protéines comme les enzymes, les albumines ou les histones accompagnant le DNA se dénaturent, de sorte qu'il n'y a plus de réaction biochimique. A cette température élevée, le spécialiste s'attend donc à n'avoir aucune amélioration, mais plutôt une diminution drastique de l'activité.

Mais le Demandeur a découvert que jusque 40°C l'activité des DNAases ne subit qu'une légère augmentation avec l'augmentation de la température. L'augmentation de l'activité s'accentue à 40°C et à partir de 45°C, fait un bond qui ne s'explique plus par une activation normale due à une hausse de température, mais qui est probablement due au fait que l'enzyme est lié, au moins partiellement, à une substance complexante, et que ce complexe est scindé ou détruit à partir de 40°C et principalement à partir de 45°C.

A la Fig. 3, la courbe marquée "55°C" montre le profil de l'évolution de l'activité des DNAases alcalines sérique chez un patient cancéreux traité par deux traitements successifs de chimiothérapie, étant entendu que l'activité des DNAases est déterminée par le procédé conforme à l'invention, avec une incubation à 55°C pendant une heure. A cette même Fig. 3, la courbe marquée "37°C" a été obtenue en déterminant l'activité des DNAases chez le même patient (et pendant la même période) en utilisant un procédé de détermination similaire, mais avec une incubation à 37°C, toutes les autres conditions étant par ailleurs égales.

En comparant les deux courbes de la Fig. 3, on observe que le procédé avec incubation à 55°C fait apparaître des variations d'activité de l'enzyme qui ne sont pas décelables à une température de 37°C. Avec le procédé à 55°C, on observe également une très nette variation de cette activité en fonction du traitement administré. Cette dernière information, essentielle pour les applications cliniques de la mesure, ne peut être obtenue que grâce à l'action de la température qui débloque le complexe enzymatique.

Le Demandeur a constaté qu'il est avantageux de conduire cette incubation à une température voisine de 55°C. En effet, à partir de 60°C, on peut observer une dénaturation de l'enzyme, qui, à partir de 63°C, est totalement détruit empêchant toute détermination.

Pour la détermination, on utilise avantageusement de 25 à 500 µl du liquide biologique, de 250 à 750 g d'acide désoxyribonucléique, du chlorure de magnésium d'une concentration dans le mélange de réaction de 0,1 à 50 mM et du chlorure de calcium d'une concentration dans le mélange de réaction de 0,01 à 3 mM.

De préférence on utilise de 400 à 600 µg d'acide désoxyribonucléique.

L'origine du DNA est d'une importance secondaire, même si les DNA de différentes provenances se distinguent d'après leur contenu en substances secondaires et d'après leur degré de polymérisation. La préférence est donnée au substrat DNA hautement polymérisé provenant des thymus de veau.

On peut choisir comme solution tampon une solution dont le pH se situe entre 7 et 9. On préférera utiliser une solution tampon qui se compose de TRIS-(tris(hydroxyméthyl)-aminométhane)-HCl. On dilue dans cette solution tampon de petites quantités de chlorure de magnésium (de préférence pour avoir une concentration comprise entre 3 à 8 mmoles dans le mélange de réaction) et de chlorure de calcium (de préférence pour avoir une concentration comprise entre 0,1 à 0,5 mmole dans le mélange de réaction).

Le mélange de réaction contenant le liquide à incuber est maintenu pendant 45 à 90 minutes à une température de 40 à 63°C. Vu que l'activité des enzymes sera mesurée en fonction du temps, il est important de mettre fin à l'activité des enzymes immédiatement après le temps d'incubation. Cela se fait par addition d'une petite quantité de substance dénaturante usuelle, de préférence, on utilise une solution d'acide perchlorique. La concentration d'acide perchlorique dans le mélange de réaction est d'au moins 0,5 M.

Les volumes donnés sont recommandés pour des travaux microanalytiques. Il va de soi que le même procédé de détermination est réalisable de façon macroanalytique avec de plus grandes quantités.

Comme indiqué plus haut, après dénaturation, les substances non solubles seront éliminées du mélange de réaction.

Suivant une première forme d'exécution du procédé de l'invention, la séparation des substances insolubles est effectuée par une ultracentrifugation à plus de 40000 g. Il a en effet été constaté qu'une centrifugation à 2000-3000 g ne suffit pas pour obtenir une séparation qui donne des valeurs d'extinction reproductibles. Il reste encore beaucoup de substances solides dans la partie liquide qui influencent de façon non contrôlée l'extinction à 260 nm. Avec une telle ultracentrifugation pendant environ 5 minutes, on obtient une séparation totale de toutes les substances solides du mélange dénaturé et de là une très bonne reproductibilité.

Suivant une variante de cette forme d'exécution du procédé, la séparation des substances insolubles est effectuée par une filtration à travers un filtre dont le diamètre des pores est de 0,3 à 0,6µm.

Des filtres à plus petits pores ralentissent trop la vitesse de filtration, et des filtres à plus grands pores ne donnent pas de séparation suffisante.

Quoique, en principe, toute membrane inerte à pores de cette dimension puisse convenir comme filtre, le Demandeur a constaté que les filtres à membranes en nitrocellulose ou en polytétrafluoroéthylène sont préférables. On peut utiliser une ou plusieurs couches de filtres et combiner les deux sortes de filtres.

Pour faciliter la filtration, il est utile de centrifuger le mélange dénaturé d'abord brièvement pour que les grands coagulats se posent au fond du récipient.

On mesure de manière connue l'extinction à 260 nm de la solution filtrée et on la compare à une solution de référence.

Pour cette première forme d'exécution du procédé, on utilise avantageusement de 100 à 500 µl du liquide biologique.

Suivant une autre forme d'exécution très avantageuse du procédé, un sel soluble dont l'anion est capable d'engendrer un processus de relargage est ajouté au mélange après l'incubation et avant la dénaturation.

Ce sel soluble dont l'anion est capable d'engendrer un processus de relargage est de préférence un sulfate. Le relargage se fait juste avant la dénaturation par addition d'une quantité de 100 à 1000 µl d'un sulfate soluble sous la forme d'une solution saturée. Pour engendrer le processus de relargage, on peut notamment utiliser tous les sulfates solubles, par exemple le sulfate de magnésium, le sulfate de zinc, le sulfate de sodium, le sulfate d'ammonium, etc.

Le Demandeur a constaté que l'utilisation du sulfate de magnésium représente un avantage supplémentaire. Du fait que le culot obtenu avec le sulfate de magnésium est plus solide, il est plus facile à manipuler. Ceci pourrait être dû au fait que le $Mg^{++}$ lie le DNA aux protéines. Par conséquent, on ajoute, de préférence, au mélange de réaction une solution saturée de sulfate de magnésium.

On peut également ajouter au mélange de réaction une solution de sulfate en dessous de la saturation mais, pour assurer une bonne reproductibilité de la méthode de dosage, il importe que, par exemple, pour le sulfate de magnésium, sa concentration dans le mélange de réaction soit au moins de 10mM.

Dans cette deuxième forme d'exécution du procédé comprenant une étape de relargage, la séparation des substances insolubles du mélange de réaction peut ensuite être effectuée avantageusement par une centrifugation "normale", c'est-à-dire une centrifugation à vitesse relativement basse (par exemple une centrifugation à environ 500 à 5000 g). En effet, grâce au processus de relargage qui précède, il n'est nullement nécessaire de procéder à une ultracentrifugation pour séparer les substances insolubles du mélange de réaction.

Les substances non solubles peuvent également être éliminées du mélange de réaction par filtration.

Pour cette deuxième forme d'exécution, on utilise de 25 à 200 µl du liquide biologique.

Quelle que soit la variante du procédé utilisé, on mesure de manière connue l'extinction à 260 nm de la solution filtrée et on la compare à une solution de référence. On prépare la solution de référence dans les mêmes conditions opératoires en ajoutant de l'EDTA avant incubation.

Les liquides biologiques susceptibles d'être testés sont notamment le sang, le liquide céphalorachidien, les liquides de ponctions notamment pleurales, péritonéales, etc., le liquide lymphatique, le suc pancréatique et le liquide biliaire, l'urine et les parties solubles séparées des particules solides d'homogénats de tissu ou de glandes.

Pour comparer la technique de l'invention avec les procédés Loiselle et Spandidos connus, on a soumis des échantillons de sérum de cinq personnes saines et quatre patients atteints d'une tumeur aux trois méthodes de détermination de l'activité des DNAases, à savoir celle de Loiselle, celle de Spandidos et celle suivant l'invention. Les conditions opératoires de ce test comparatif sont données dans le tableau récapitulatif suivant.

TABLEAU RECAPITULATIF

| Méthode | I Loiselle | II Spandidos | III "Méthode de l'invention" | |
|---|---|---|---|---|
| | | | Exécution 1 | Exécution 2 |
| Type de DNA utilisé | sperme de saumon | thymus de veau | thymus de veau | thymus de veau |
| Quantité de DNA/test | 1200 $\mu$g | 100 $\mu$g | 500 $\mu$g | 500 $\mu$g |
| Tampon Tris-HCl | 0,05 M | 0,1 M | 0,1 M | 0,1 M |
| pH | 7,6 | 8 | 8 | 8 |
| $MgCl_2$ | 342 mM | – | 5 mM | 5 mM |
| $CaCl_2$ | 16,2 mM | – | 0,25 mM | 0,25 mM |
| Vol. de sérum | 500 $\mu$l | 25 $\mu$l | 200 $\mu$l | 100 $\mu$l |
| Sucrose 0,35 M | 700 $\mu$l | – | – | – |
| Vol. final d'incub. | 3,6 ml | 1 ml | 1 ml | 1 ml |
| Température d'incub. | 37°C | 25°C | 55°C | 55°C |
| Temps d'incubation | 180 min | 15 min | 60 min | 60 min |
| Relargage par sulfate soluble saturé ($MgSO_4$) | – | – | – | 500 $\mu$l |
| Acide perchlorique (PCA) | 4 M | 1,5 M | 1,5 M | 2,25 M |
| Volume de PCA | 1,2 ml | 2 ml | 2 ml | 1,5 ml |
| Centrifugation | 2800 g | 3000 g | 45000 g | 1000 g |

Exécution 1.-

La séparation des substances insolubles du mélange est effectuée par ultracentrifugation.

Exécution 2.-

Suivant cette exécution du procédé, du sulfate de magnésium est ajouté au mélange de réaction après l'incubation et avant la dénaturation, ensuite la séparation des substances insolubles est effectuée par une centrifugation "normale".

Le tableau II montre les résultats des mesures spectrophotométriques faites à 260 nm pour les trois méthodes par rapport aux blancs respectifs.

| Echantillon | LOISELLE | SPANDIDOS | METHODE SELON L'INVENTION | |
| | | | Exécu-tion 1 | Exécu-tion 2 |
| | 37 °C | 25 °C | 55 °C | 55 °C |
|---|---|---|---|---|
| Normal | | | | |
| 1 | 115 | −63 | 1498 | 1358 |
| 2 | 80 | −106 | 1211 | 919 |
| 3 | 92 | −57 | 1414 | 991 |
| 4 | 37 | −16 | 396 | 219 |
| 5 | 55 | −78 | 865 | 608 |
| Pathologique | | | | |
| 6 | 21 | −144 | 502 | 370 |
| 7 | ILLIS | ILLIS | 172 | 120 |
| 8 | 98 | −39 | 1486 | 1150 |
| 9 | ILLIS | ILLIS | 39 | 20 |

ILLIS = illisible.

Les chiffres sont donnés en millièmes de densité optique.

Ce tableau montre que la méthode Spandidos ne donne que des résultats négatifs non interprétables. D'autre part, la diminution de l'activité des DNAses due aux tumeurs chez le patient cancéreux peut être telle qu'elle n'est plus décelable par la méthode de Loiselle. Quand la mesure d'extinction n'est que de quelques dizaines de millièmes de densité optique au départ et que l'on désire examiner si un traitement est positif par une diminution de la densité optique, cela n'est plus possible avec la méthode Loiselle. De plus, dans deux cas pathologiques sur quatre, la lecture était illisible probablement due à l'interférence des substances solides en suspension. Enfin les chiffres dans le tableau étant des moyennes de plusieurs expériences du même échantillon, on a pu constater une divergence de résultats pour les procédés connus et une reproductibilité fiable pour le procédé de l'invention.

Le nouveau procédé est particulièrement utile pour le dépistage de tumeurs et des métastases, pour contrôler le pronostic d'un traitement de tumeurs, pour suivre l'évolution clinique de malades porteurs des tumeurs traitées ou non traitées, et pour confirmer le diagnostic.

Le procédé de l'invention peut être effectué par le personnel habituel d'un laboratoire d'analyse médicale et il donne des résultats fiables et bien reproductibles.

Le test effectué avec le liquide biologique des patients est répété plusieurs fois avant, pendant et après le traitement thérapeutique et à des intervalles réguliers pour la détection d'une éventuelle reprise tumorale. La détermination de l'activité des DNAses avant, et pendant le traitement des tumeurs malignes rend possible le contrôle du déroulement et des résultats de la thérapie.

Les exemples suivants illustreront mieux l'invention. Les exemples 1 et 2 sont effectués suivant la première forme d'exécution du procédé et les exemples 3 à 8 suivant la deuxième forme d'exécution.

EXEMPLE 1.-

On ajoute 500 µg de DNA (de thymus de veau) à 800 µl d'une solution tampon à pH 8 (0,1 M TRIS/HCl) contenant 5 mmoles de MgCl₂ et 0,25 mmole de CaCl₂. Dans le milieu de réaction, 200 µl de sérum de sang sera incubé pendant 60 minutes à 55°C. Ensuite, on ajoute 2 ml d'une solution d'acide perchlorique glacée (1,5 M) et, après une courte agitation, on le laisse reposer dans la glace pendant 30 minutes, ensuite la solution sera centrifugée à 3000 g pendant ~10 minutes.

On sépare le mélange dénaturé sur un filtre en nitrocellulose ayant un diamètre des pores de 0,45 µm. La solution filtrée est mesurée au spectrophotomètre UV où la mesure se fait à 260 nm. L'extinction est comparée à une solution de référence préparée avec le même sérum de sang dans les mêmes conditions opératoires, mais avec de l'EDTA (acide édétique). L'extinction mesurée est 1486. Cette extinction est exprimée en millièmes de densité optique; il en est de même dans les exemples suivants.

EXEMPLE 2.-

On ajoute 500µg de DNA (de thymus de veau) à 800µl d'une solution tampon à pH 8 (0,1 M TRIS/HCl) contenant 5 mmoles de MgCl₂ et 0,25 mmole de CaCl₂. Dans le milieu de réaction 200 µl de ponction pleurale seront incubés pendant 60 minutes à 55°C. Ensuite, on ajoute 2 ml d'une solution d'acide perchlorique glacée (1,5 M) et, après une courte agitation, on le laisse reposer dans la glace pendant 30 minutes, ensuite la solution sera centrifugée à 3000 g pendant ~10 minutes.

On sépare le mélange dénaturé par une ultracentrifugation à 45000 g pendant 5 minutes. La solution surnageante est mesurée au spectrophotomètre UV à 260 nm. L'extinction est comparée à une solution de référence préparée des mêmes ponctions pleurales dans les mêmes conditions opératoires, mais avec de l'EDTA. L'extinction trouvée est 1150.

EXEMPLE 3.-

On ajoute 500 µg de DNA (de thymus de veau) à 900 µl d'une solution tampon à pH 8 (0,1 M TRIS/HCl) contenant 5 mmoles de MgCl₂ et 0,25 mmole de CaCl₂. Dans le milieu de réaction, 100 µl de sérum de sang sera incubé pendant 60 minutes à 55°C. Ensuite, on ajoute 500 µl d'une solution saturée de sulfate de magnésium. On dénature le mélange de réaction avec 1,5 ml d'une solution d'acide perchlorique glacée (2,25 M) et après une courte agitation on le laisse reposer dans la glace pendant 30 minutes, ensuite la solution sera centrifugée à 1000 g pendant ~20 minutes. En procédant de la sorte, le mélange dénaturé ne contient plus de substances solides qui dérangeraient la reproductibilité de la mesure d'extinction. La densité optique du liquide restant est mesurée à 260 nm. L'extinction est comparée à une solution de référence préparée du même sérum de sang dans les mêmes conditions opératoires, mais avec de l'EDTA. L'extinction trouvée est 991.

EXEMPLE 4.-

L'exemple 3 est répété avec un autre échantillon de sérum de sang et, après l'incubation, on ajoute dans le milieu de réaction 500 µl d'une solution saturée de sulfate de zinc. L'extinction mesurée est 821.

EXEMPLE 5.-

L'exemple 3 est répété avec un autre échantillon de sérum de sang et, après l'incubation, on ajoute dans le milieu de réaction 200 µl d'une solution saturée de sulfate de sodium. L'extinction mesurée est 661.

EXEMPLE 6.-

L'exemple 3 est répété avec un autre échantillon de sérum de sang et, après l'incubation, on ajoute dans le milieu de réaction 200 µl d'une solution saturée de sulfate d'ammonium. L'extinction mesurée est 694.

EXEMPLE 7.-

L'exemple 3 est répété avec une autre échantillon de sérum de sang et, après l'incubation, on ajoute dans le milieu de réaction 500 µl d'une solution saturée de sulfate de magnésium. L'extinction mesurée est 219.

EXEMPLE 8.-

L'exemple 7 est répété, mais après l'incubation, on ajoute dans le milieu de réaction 500 µl d'une solution non saturée de sulfate de magnésium. La concentration du sulfate de magnésium dans le mélange obtenu est de 70 mM. L'extinction mesurée est 200.

0 224 463

## Revendications

1.- Procédé de détermination de l'activité des désoxyribonucléases dans des liquides biologiques, suivant lequel on ajoute le liquide biologique à examiner à un milieu de réaction composé d'une solution tampon, d'acide désoxyribonucléique, d'un sel soluble de magnésium et d'un sel soluble de calcium, on incube le mélange de réaction ainsi obtenu pendant un temps fixe prédéterminé, on le dénature, on sépare les substances insolubles du mélange de réaction et on soumet le liquide restant à une mesure spectrophotométrique à 260 nm par rapport à une solution de référence, caractérisé en ce qu'on incube le mélange de réaction à une température de 40 à 63°C.

2.- Procédé suivant la revendication 1, caractérisé en ce que le mélange de réaction est maintenu pendant 45 à 90 minutes à une température de 45 à 63°C.

3.- Procédé suivant l'une quelconque des revendications précédentes, caractérisé en ce que le mélange de réaction est maintenu pendant 50 à 70 minutes à une température de 55 à 60°C.

4.- Procédé suivant l'une quelconque des revendications précédentes, caractérisé en ce que la solution tampon a un pH de 7 à 9.

5.- Procédé suivant l'une quelconque des revendications précédentes, caractérisé en ce que la solution tampon consiste en une solution aqueuse de chlorhydrate de tris-(hydroxyméthyl)-aminométhane.

6.-Procédé suivant l'une quelconque des revendications précédentes, caractérisé en ce que le produit de dénaturation est l'acide perchlorique et que sa concentration dans le mélange de réaction est d'au moins 0,50 M.

7.- Procédé suivant l'une quelconque des revendications précédentes, caractérisé en ce qu'après avoir dénaturé le mélange, on le refroidit à une température proche de 0°C et on le maintient à cette température pendant au moins 30 minutes.

8.- Procédé suivant l'une quelconque des revendications précédentes, caractérisé en ce qu'on utilise de 25 à 500 µl du liquide biologique, de 250 à 750 µg d'acide désoxyribonucléique, du chlorure de magnésium d'une concentration dans le mélange de réaction de 0,1 à 50 mM, et du chlorure de calcium d'une concentration dans le mélange de réaction de 0,01 à 3 mM.

9.- Procédé suivant la revendication 8, caractérisé en ce qu'on utilise de 25 à 500 µl du liquide biologique, de 400 à 600 µg d'acide désoxyribonucléique, du chlorure de magnésium d'une concentration dans le mélange de réaction de 3 à 8 mM, et du chlorure de calcium d'une concentration dans le mélange de réaction de 0,1 à 0,5 mM.

10.- Procédé suivant l'une quelconque des revendications précédentes, caractérisé en ce que la solution de référence est préparée dans les mêmes conditions opératoires que le mélange à mesurer mais non incubée.

11.- Procédé suivant l'une quelconque des revendications 1 à 9, caractérisé en ce que la solution de référence est préparée dans les mêmes conditions opératoires que le mélange à mesurer mais contient en plus un inhibiteur des DNAases.

12.- Procédé suivant la revendication 11, caractérisé en ce que l'inhibiteur des DNAases est l'acide édétique.

13.- Procédé suivant l'une quelconque des revendications précédentes, caractérisé en ce que la séparation des substances insolubles est effectuée par une filtration à travers un filtre dont le diamètre des pores est de 0,30 à 0,60 µm.

14.- Procédé suivant la revendication 13, caractérisé en ce qu'on fait précéder la filtration d'une centrifugation.

15.- Procédé suivant l'une quelconque des revendications 1 à 12, caractérisé en ce que la séparation des substances insolubles est effectuée par une ultracentrifugation à plus de 40.000 g.

16.- Procédé suivant l'une quelconque des revendications précédentes, caractérisé en ce qu'on utilise de 100 à 500 µl du liquide biologique.

17.- Procédé suivant l'une quelconque des revendications 1 à 12, caractérisé en ce qu'après l'incubation et avant la dénaturation, on ajoute dans le mélange de réaction un sel soluble dont l'anion est capable d'engendrer un processus de relargage.

18.- Procédé suivant la revendication 17, caractérisé en ce que le sel soluble dont l'anion est capable d'engendrer un processus de relargage est un sulfate.

19.- Procédé suivant la revendication 18, caractérisé en ce que le sulfate est ajouté au mélange de réaction sous la forme d'une solution saturée.

20.- Procédé suivant l'une quelconque des revendications 18 et 19, caractérisé en ce que le sulfate est choisi parmi le sulfate de magnésium, le sulfate de zinc, le sulfate de sodium et le sulfate d'ammonium.

21.- Procédé suivant la revendication 20, caractérisé en ce que le sulfate est le sulfate de magnésium et que sa concentration dans le mélange de réaction est d'au moins 10 mM.

22.- Procédé suivant l'une quelconque des revendications 17 à 21, caractérisé en ce qu'on sépare les substances insolubles du mélange de réaction par centrifugation.

9

23.- Procédé suivant la revendication 22, caractérisé en ce que la séparation des substances insolubles est effectuée par une centrifugation à environ 500 à 5000 g.

24.- Procédé suivant l'une quelconque des revendications 17 à 23, caractérisé en ce qu'on utilise de 25 à 200 l du liquide biologique.

25.- Procédé suivant l'une quelconque des revendications précédentes, caractérisé en ce que le liquide biologique est choisi parmi le sang, le liqude céphalorachidien, les liquides de ponctions notamment pleurales, péritonéales, etc., le liquide lymphatique, le suc pancréatique et le liquide biliaire, l'urine et les parties solubles séparées des particules solides d'homogénats de tissu ou de glandes.

26.- Utilisation du procédé suivant l'une quelconque des revendications précédentes, pour le dépistage de tumeurs et des métastases.

27.- Utilisation du procédé suivant l'une quelconque des revendications 1 à 25, pour contrôler le pronostic d'un traitement de tumeurs, pour suivre l'évolution clinique de malades porteurs des tumeurs traitées et non traitées, et confirmer le diagnostic.

0224463

Fig. 1.

Fig. 2

0224463

Fig. 3.

55°C

37°C

(IU/L) ĎĎŇŇŎMŐŇ ALCALINE/ml SERUM

40000

30000

20000

10000

To  5  10  15  20  25  30  35  40  45  50  55  60  65  70  75  80  85  90  95  100  140  JOURS

CHIMIOTHERAPIE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.⁴) |
|---|---|---|---|
| A | FR-A-2 242 685 (BEHRINGWERKE AG) <br> * Page 3, lignes 13-40; page 4, lignes 1-5; page 6, exemple 6, lignes 26-40 * <br><br> --- | 1,4,5, 8,9 | C 12 Q 1/34 <br> C 12 Q 1/68 |
| A | CHEMICAL ABSTRACTS, vol. 86, no. 17, 25 avril 1977, pages 157-158, résumé no. 116483q, Columbus, Ohio, US; L. SNECKIENE et al.: "Determination of the activity of acid nucleases", & METODY BIOKHIM., MATER. S'EZDU BIOKHIM. LIT. SSR, 2ND 1975, 191-2 <br><br> --- | | |
| A | CHEMICAL ABSTRACTS, vol. 94, no. 15, 13 avril 1981, page 10, résumé no. 114161z, Columbus, Ohio, US; H.S. TAPER et al.: "Increase in nuclease activity as a possible means for detecting tumor cell sensitivity to anticancer agents", & CANCER (PHILADELPHIA) 1981, 47(3), 523-9 <br><br> ---      -/- | | **DOMAINES TECHNIQUES RECHERCHES (Int. Cl.⁴)** <br><br> C 12 Q |

Le présent rapport de recherche a été établi pour toutes les revendications

| Lieu de la recherche <br> LA HAYE | Date d'achèvement de la recherche <br> 29-01-1987 | Examinateur <br> MEYLAERTS H. |
|---|---|---|

## DOCUMENTS CONSIDERES COMME PERTINENTS

Page  2

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS, vol. 71, no. 3, 21 juillet 1969, page 61, résumé no. 10165n, Columbus, Ohio, US; H.S. TAPER: "Histochemical detection of alkaline deoxyribonuclease", & ANN. HISTOCHIM. 1968, 13(4), 301-17 | | |
| | --- | | |
| A | H.U. BERGMEYER et al.: "Methoden der enzymatischen Analyse", vol. 1, 3ème édition, page 475, Verlag Chemie, Weinheim/Bergstr., DE; * Page 475 * | 1,20 | |
| | ----- | | |

DOMAINES TECHNIQUES RECHERCHES (Int. Cl.4)

Le présent rapport de recherche a été établi pour toutes les revendications

| Lieu de la recherche LA HAYE | Date d'achèvement de la recherche 29-01-1987 | Examinateur MEYLAERTS H. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82